(19) Europäisches Patentamt
European Patent Office
Office européen des brevets

(11) Publication number: **0 411 494 A2**

(12) **EUROPEAN PATENT APPLICATION**

(21) Application number: **90114444.4**

(22) Date of filing: **27.07.90**

(51) Int. Cl.[5]: **C07D 457/02**, C07D 457/04, C07D 457/06

(30) Priority: **04.08.89 IT 2145689**

(43) Date of publication of application: **06.02.91 Bulletin 91/06**

(84) Designated Contracting States: **BE DE ES FR GB**

(71) Applicant: **POLI INDUSTRIA CHIMICA S.p.A.**
**Piazza Agrippa, 1**
**I-20141 Milano(IT)**

(72) Inventor: **Poli, Stefano**
**Piazza Agrippa 1**
**I-20141 Milano(IT)**
Inventor: **Coppi, Germano**
**Piazza Agrippa 1**
**I-20141 Milano(IT)**
Inventor: **Del Corona, Lucio**
**Piazza Agrippa 1**
**I-20141 Milano(IT)**

(74) Representative: **Minoja, Fabrizio**
**Studio Consulenza Brevettuale Via Rossini, 8**
**I-20122 Milano(IT)**

(54) **Ergoline derivatives having dopaminergic activity.**

(57) Ergoline derivatives having dopaminergic activity, the processes for the preparation thereof and pharmaceutical compositions containing them.

EP 0 411 494 A2

# ERGOLINE DERIVATIVES HAVING DOPAMINERGIC ACTIVITY

The present invention relates to compounds of formula (I) :

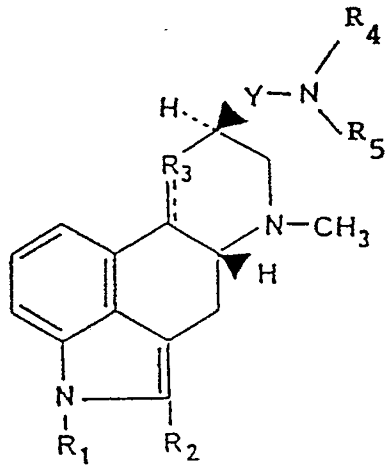

(I)

wherein :

$R_1$ is hydrogen or $C_1$-$C_6$ alkyl;

$R_2$ is hydrogen, chlorine, bromine or iodine;

$R_3$ is CH or $CH_2$, depending on the presence or absence of the double bond represented by the dotted line;

$R_4$ is hydrogen or $C_1$-$C_6$ alkyl;

$R_5$ is hydrogen or $C_3$-$C_6$ cycloalkyl, (alkylcycloalkyl)aminoethyl, wherein alkyl and cycloalkyl have the above mentioned meanings;

Y is $CH_2$ or C=O.

Preferably, $R_1$ is hydrogen or methyl, $R_2$ is hydrogen or bromine, $R_4$ is hydrogen or methyl, $R_5$ is cyclopropyl, cyclobutyl, cyclopentyl, cyclohexyl and (cyclopropylmethyl)aminoethyl.

The present invention also relates to the pharmaceutically acceptable acid salts of the compounds of formula (I).

In recent years, different lysergic acid derivatives have been synthesized in order to find out dopaminergic agents alternative to Ergot alkaloids obtained by fermentation or extraction from Claviceps purpurea cultures. The main aim of this search is to provide molecules which could selectively act in dopaminergic receptors, reducing the toxic side-effects characteristic of this class of substances.

Ergoline derivatives of formula (I) satisfactorily fulfil said requirements, since they proved to have very effective antihypertensive and antihyperprolactinemic activities and in principle better activity on Central Nervous System than such known drugs as dihydroergocristine, dihydroergocryptine, bromocryptine, nicergoline, metergoline, cabergoline, and methysergid.

More particularly, the compounds identified in the examples below by the codes P 1427 and P 1428, respectively N-methyl-N-cyclopropyl-9,10-didehydro-6-methylergoline-8β-methanamine (I, $R_1$ = H, $R_2$ = H, $R_3$ = CH, $R_4$ = $CH_3$, $R_5$ = ◁ , Y = $CH_2$) and the corresponding 9,10-dihydroderivative (I as above, with $R_3$ = $CH_2$) have high hypotensive and antiserotonin activities; the same compounds and P1426 or N-methyl-N-cyclopropyl-9,10-didehydro-6-methyl-ergoline-8β-carboxyamide ((I, $R_1$ = $R_2$ = H, $R_3$ = CH, $R_4$ = $CH_3$, $R_5$ = ◁ , Y = CO) show an interesting antihyperprolactinemic activity; P 1426 has a remarkable activity on C.N.S.

Compounds of formula (I) generally have a marked dopaminergic action at the C.N.S. level.

The results from the pharmacological tests carried out with the compounds of the invention are summarized in the following Tables.

TABLE I -

| Stereotypy test in the rat | | |
| --- | --- | --- |
| Compound | Dose mg/kg i.p. | Mean score |
| P 1426 | 1.25 | 2.37 |
| P 1424 | 5 | 2.12 |
| P 1428 | 20 | 2.25 |
| α-Bromocryptine | 10 | 2.25 |
| Dihydroergocristine | 10 | 2.37 |

In the locomotor activity in the mouse, all the compounds of the invention displayed a remarkable inhibition, thereby proving the C.N.S. dopaminergic activity. The results are reported in Table II.

TABLE II

| Compound | Dose mg/kg i.p. | % inhibition |
| --- | --- | --- |
| P 1424 | 5 | 70.0 |
| P 1426 | 1.25 | 83.2 |
| P 1427 | 20 | 92.7 |
| P 1428 | 20 | 87.2 |
| α-Bromocryptine | 10 | 78.7 |
| Dihydroergocristine | 10 | 47.7 |
| Dihydro-α-ergocryptine | 20 | 74.3 |

All the compounds of formula (I) have antihypertensive activity on Spontaneously hypertensive rats (SHR), as clearly shown by the results reported in the following Table III.

TABLE III

| Compound | Mean arterial pressure % reduction | |
| --- | --- | --- |
| | dose = 1 mg/kg i.p. | dose = 3 mg/kg i.p. |
| P 1424 | 19.8 | 26.7 |
| P 1427 | 35.6 | 32.4 |
| P 1428 | 28.8 | 30.7 |
| Methylergometrine | 15.7 | 29.4 |
| Dihydro-α-ergocryptine | 18.3 | 29.0 |
| Dihydroergocristine | 18.1 | 32.0 |

Most of the compounds of formula (I) have a marked adrenolytic activity in the rat seminal vesicle test in vitro . The results are shown in Table IV.

TABLE IV

| Compound | $ED_{50}$ (mcg/ml) |
|---|---|
| P 1424 | 2.21 |
| P 1426 | 1.58 |
| P 1427 | 0.70 |
| P 1428 | 1.42 |
| Methysergid | 2.48 |

All the compounds of the invention have a marked antiserotonin activity in vitro test on isolated uterus of female rat. The results are reported in Table V.

TABLE V

| Compound | $ED_{50}$ (mcg/ml) |
|---|---|
| P 1424 | 0.080 |
| P 1426 | 0.004 |
| P 1427 | 0.004 |
| P 1428 | 0.035 |
| Dihydro-α-ergocryptine | 0.035 |
| Dihydroergocristine | 0.007 |
| α-Bromocryptine | 0.470 |

All compounds (I) have a marked antihyper prolactinemic activity in the reserpinized rat. The results are reported in Table VI.

TABLE VI

| Compound | Plasmatic prolactin % reduction | |
|---|---|---|
| | After 1 hour | After 2 hours |
| | (dose 0.25 mg/kg os) | (dose 0.50 mg/kg os) |
| P 1424 | 74.0 | |
| P 1426 | 86.4 | 88.9 |
| P 1427 | 67.2 | 86.6 |
| P 1428 | 61.8 | 79.4 |
| Dihydro-α-ergocryptine | 52.4 | 66.1 |
| Dihydroergocristine | 47.4 | |
| α-Bromocryptine | 78.2 | 80.9 |

Moreover, all the compounds (I) have an effective antiaggregating activity on the in vitro guinea pig platelet aggregation induced by ADP. The results are reported in Table VII.

TABLE VII

| Compound | Dose mcg/ml | Platelet aggregation % reduction |
|---|---|---|
| P 1426 | 40 | 25 |
| P 1427 | 40 | 25 |
| P 1428 | 20 | 25 |
| Dihydro-α-ergocryptine | 40 | 25 |
| Dihydroergocristine | 40 | 25 |
| α-Bromocryptine | 80 | 25 |

The compounds of the invention have an acute toxicity by the oral route in the mouse ranging from 400 to 1,600 mg/kg.

All the compounds of the invention exhibit depression signs at the level of C.N.S. which can be related to the dopaminergic action.

The present invention also relates to all the industrial aspects connected to the use of compounds of formula (I) for the treatment of Parkinson's disease, cephalalgias, depressions, hyperprolactinemias, hypertension and peripheral and cerebral vascular insufficiencies.

Therefore, the present invention also relates to pharmaceutical compositions containing compounds (I) as the active ingredients, suitably formulated for the oral administration, in form of hard or soft gelatin capsules, dragees, tablets, granulates, drops, syrups, sustained-release forms and for the parenteral administration, in form of solutions or freeze-dried vials.

The pharmaceutical compositions of the invention are prepared according to conventional techniques, using compatible, pharmaceutically acceptable carriers and excipients, and could also contain, in combination, other active ingredients having complementary or anyhow useful activity.

The dose will vary depending on the age, body weight and condition of the patient, as well as on the nature and severity of the disorder; generally it will range from 1 to 100 mg of active ingredient, preferably from 2 to 20 mg for the oral forms; from 0.1 to 20 mg, preferably from 0.2 to 5 mg, for the parenteral forms.

The compounds of the invention can be prepared according to conventional methods, starting from D-lysergic acid, for instance according to the reaction scheme A.

SCHEME A

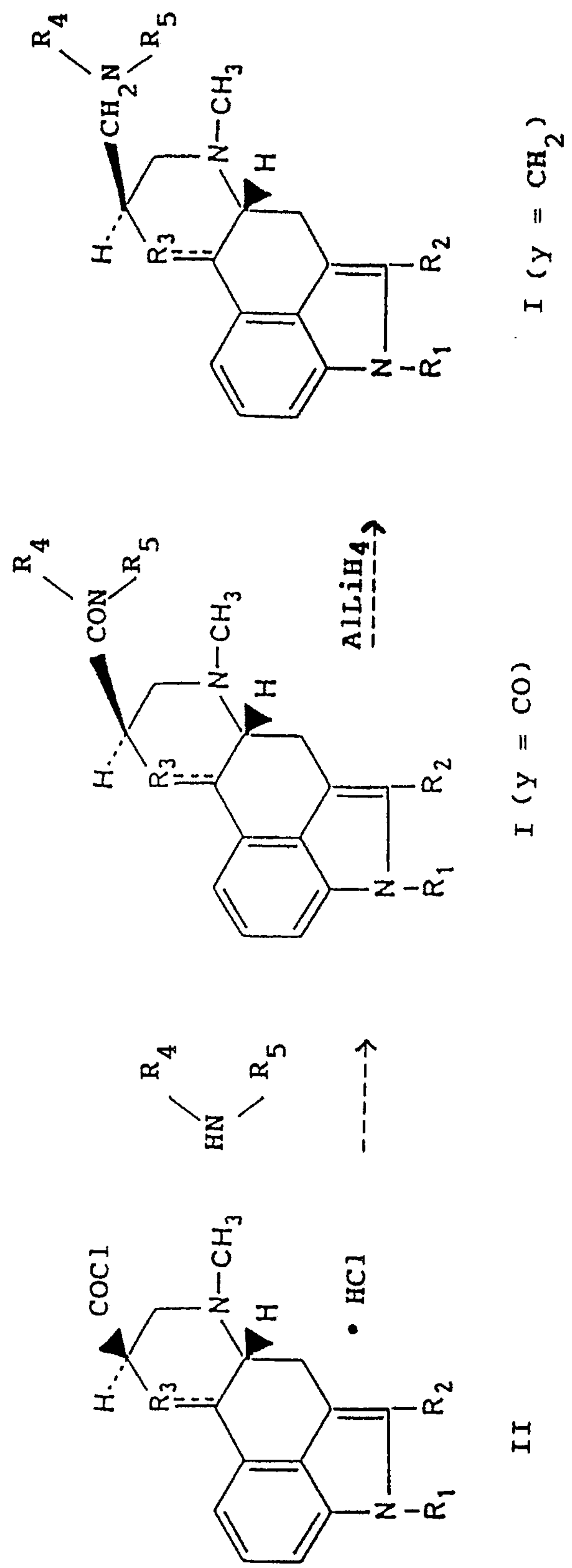


II

I (y = CO)

I (y = $CH_2$)

Chloride hydrochloride (II) (GB pat. 1.277.006) is condensed with the corresponding amines in solvents like chloroform, methylene chloride, tetrahydrofuran, dioxane or mixtures thereof, in the presence of organic bases like triethylamine, to give the corresponding amides I (Y = CO) which are then treated with $AlLiH_4$ in tetrahydrofuran, sodium borohydride and calcium chloride in alcohols or sodium dihydrobismethoxyethoxyaluminate to give corresponding compounds (I) (Y = $CH_2$).

The following non limiting examples illustrate the invention in more detail.

## EXAMPLE 1

N-Methyl-N-ciclopropyl-9,10-dihydro-D-lysergamide hydrochloride (P 1452, I, $R_1$ = $R_2$ = H, $R_3$ = $CH_2$, $R_4$ = $CH_3$, $R_5$ = ◁ , Y = CO)

24.2 g (0.094 mole) of 9,10-dihydro-D-lysergyl-chloride hydrochloride are added in portions to a solution of 7.95 (0.074 mole) of methylcyclopropylamine and 35 ml (0.25 mole) of triethylamine in 300 ml of chloroform and 100 ml of dioxane at 0 °C. When the addition is completed, the reaction mixture is stirred for 30 min., washed with water, then with diluted ammonium hydroxide and evaporated under vacuum. The residue is dissolved in some chloroform and eluted through a column containing FLORISIL with 95:5 chloroform-methanol. From the fractions containing the product, by evaporation and trituration with ethyl acetate, 11.5 g (47.8%) of the base are obtained, m.p. 168-171 °C, which are transformed into the hydrochloride by treatment with alcoholic hydrochloric acid.
M.p. 227-230 °C.
Elemental analysis for $C_{20}H_{23}N_3O.HCl$
Calc. : H% = 6.75 C% = 67.11 N% = 11.80
Found: H% = 6.81 C% = 67.02 N% = 11.71

## EXAMPLE 2

2-Bromo-N-methyl-N-ciclopropyl-D-lysergamide (P 1455, $R_1$ = H, $R_2$ = Br, $R_3$ = CH, $R_4$ = $CH_3$, $R_5$ = ◁ , Y = CO)

20 g of D-bromolysergic acid (obtained in a 60% yield by bromination of methyl 9,10-dihydrolysergate and subsequent saponification of the obtained ester by means of sodium hydroxide) are suspended in 200 ml of acetonitrile, the suspension is cooled to -10 °C and added with 80 ml of phosphorus trichloride, then with 15.43 g of phosphorus pentachloride in 4 portions, during 20 min.. After 1.5 hours at -5 °C, the mixture is diluted with 500 ml of chilled ethyl ether, stirred for some minutes, then filtered, washing with ether, and dried at 40 °C under vacuum to give 21 g (91.4%) of D-bromolysergyl chloride hydrochloride.

8.06 g (0.02 mole) of this compound are added in portions at 0 °C to a solution of 2.15 g (0.02 mole) of methylcyclopropylamine hydrochloride and 11.8 ml of triethylamine in 150 ml of chloroform and 50 ml of dioxane. After 30 min. the reaction mixture is poured into water, the organic phase is evaporated, washed with diluted ammonium hydroxide and evaporated under vacuum. The residue is taken up into some chloroform and left in freezer overnight, to give 6.5 g (81%) of the base. M.p. 114-115 °C (hydrochloride m.p. 278 °-280 °C).
Elemental analysis for $C_{20}H_{22}BrN_3O$
Calc. : H% = 5.53 C% = 59.97 N% = 10.54
Found: H% = 5.44 C% = 59.88 N% = 10.52

## EXAMPLE 3

N-[2-(N-Methyl-N-cyclopropyl)aminoethyl)]-9,10-dihydro-D-lysergamide (P 1457, $R_1$ = $R_2$ = H, $R_3$ = $CH_2$, $R_4$ = H,

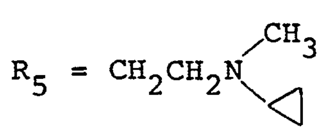

, Y = CO);

8.125 g (0.025 mole) of 9,10-dihydrolysergyl chloride hydrochloride are added to a solution of 2.65 g (0.025 mole) of N-methyl-N-cyclopropylethylene diamine in 150 ml of chloroform and 50 ml of dioxane at 0° C. After 30 min. at room temperature, the reaction mixture is poured into water, the organic phase is separated, washed with diluted ammonium hydroxide and concentrated under vacuum. The residue is dissolved in hot ethanol and the solution is left to stand overnight. The crystallized product is filtered and dried to give 6.17 g (67.4%) of the base. M.p. 188°-190° C.
Elemental analysis for $C_{22}H_{30}N_4O$
Calc. : H% = 8.25 C% = 72.09 N% = 15.28
Found: H% = 8.31 C% = 72.00 N% = 15.21

## EXAMPLE 4

N-Methyl-N-cyclopropyl-2-bromo-9,10-dihydro-D-lysergylamine (P 1464, $R_1$ = H, $R_2$ = Br, $R_3$ = $CH_2$, $R_4$ = $CH_3$, $R_5$ = ⊲ , Y = $CH_2$)

A solution of 3.3 g (0.008 mole), of N-methyl-N-cyclopropyl-2-bromo-9,10-dihydro-D-lysergamide in 50 ml of THF is added to a suspension of 0.47 g (0.012 mole) of lithium aluminium hydride in THF at 5°-10° C. The reaction mixture is refluxed for 2 hours, poured into ice, saturated with sodium hydroxide and the organic layer is separated. After concentration under vacuum, the residue is dissolved in hot ethyl acetate, petroleum ether is added until turbidity and the mixture is left to crystallize, to give 2.55 g (78%) of a product melting at 219-220° C. By addition of alcoholic hydrochloric acid, the dihydrochloride is obtained. M.p.> 300°.
Elemental analysis for $C_{20}H_{26}BrN_3$
Calc. : H% = 6.74 C% = 61.81 N% = 10.87
Found: H% = 6.66 C% = 61.63 N% = 10.78

Following the same procedures, starting from the appropriate compounds, the products listed in Table VIII were obtained.

**TABLE VIII**

Compound I

| P | FORMULA | $R_1$ | $R_2$ | $R_3$ | $R_4$ | $R_5$ | Y | M.p.°C |
|---|---|---|---|---|---|---|---|---|
| 1424 | $C_{22}H_{28}N_4O$ | H | H | CH | H | 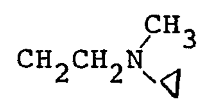 | CO | 140-142 |
| 1426 | $C_{20}H_{23}N_3O.C_4H_4O_4$ | H | H | HC | $CH_3$ | 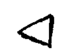 | CO | 214-215 |
| 1427 | $C_{20}H_{25}N_3$ | H | H | HC | $CH_3$ | 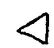 | $CH_2$ | 202-205 |
| 1428 | $C_{20}H_{27}N_3.2HCl$ | H | H | $CH_2$ | $CH_3$ | 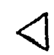 | $CH_2$ | 218-220 |
| 1451 | $C_{21}H_{29}N_3O$ | $CH_3$ | H | $CH_2$ | $CH_3$ | 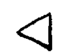 | $CH_2$ | 86-88 |
| 1456 | $C_{19}H_{24}N_3O$ | H | H | $CH_2$ | H | 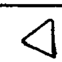 | CO | 280-282 |
| 1458 | $C_{19}H_{26}N_3.2HCl$ | H | H | $CH_2$ | H | 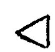 | $CH_2$ | 300 |
| 1459 | $C_{20}H_{25}BrN_3$ | H | Br | HC | $CH_3$ | 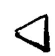 | $CH_2$ | 190-192 |

**TABLE VIII- continued -**

Compound I

| P | FORMULA | $R_1$ | $R_2$ | $R_3$ | $R_4$ | $R_5$ | Y | M.p.°C |
|---|---|---|---|---|---|---|---|---|
| 1460 | $C_{23}H_{29}N_4O$ | H | H | HC | H | 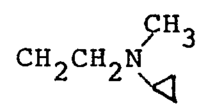 | CO | 170-171 |
| 1461 | $C_{23}H_{33}N_4$ | H | H | $CH_2$ | H | 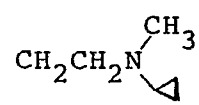 | $CH_2$ | 154-155 |
| 1462 | $C_{23}H_{29}BrN_4O$ | H | Br | HC | H | 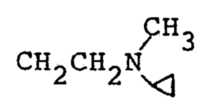 | CO | 180-182 |
| 1463 | $C_{21}H_{25}BrN_3O$ | H | Br | $CH_2$ | $CH_3$ | 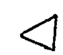 | CO | 232-233 |
| 1465 | $C_{23}H_{30}BrN_4O$ | H | Br | $CH_2$ | H | 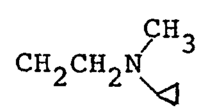 | CO | 223-225 |

## Claims

1. Compounds of formula (I) :

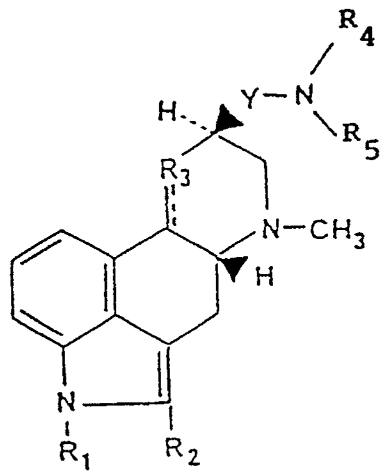

(I)

wherein :
$R_1$ is hydrogen or $C_1$-$C_6$ alkyl;
$R_2$ is hydrogen, chlorine, bromine or iodine;
$R_3$ is CH or $CH_2$, depending on the presence or absence of the double bond represented by the dotted line;
$R_4$ is hydrogen or $C_1$-$C_6$ alkyl;
$R_5$ is hydrogen or $C_3$-$C_6$ cycloalkyl, (alkylcycloalkyl)aminoethyl, wherein alkyl and cycloalkyl have the above mentioned meanings;
Y is $CH_2$ or C=O, and the pharmaceutically acceptable sats thereof.

2. Compounds as claimed in claim 1, wherein $R_1$ is huydrogen or methyl.

3. Compounds as claimed in claims 1 or 2, wherein $R_2$ is hydrogen or bromine.

4. Compounds as claimed in any one of claims 1 to 3; wherein $R_4$ is hydrogen or methyl.

5. Compounds as claimed in any one of claims 1 to 4, wherein $R_5$ is cyclopropyl, cyclobutyl, cyclopentyl, cyclohexyl or (cyclopropylmethyl)aminoethyl.

6. Pharmaceutical compositions containing as the active ingredient one compound as claimed in claims 1-5 or a pharmaceutically acceptable salt thereof in admixture with a pharmaceutically acceptable carrier or excipient.

7. Pharmaceutical compositions as claimed in claim 6, in form of tablets, hard or soft gelatin capsules, sachets, syrup, drops, vials, at doses ranging from 0.1 to 20 mg of the active ingredient.

8. A compound as claimed in claims 1 to 5 or a pharmaceutically acceptable salt thereof, for use as a therapeutic agent.

9. The use of one compound as claimed in claims 1 to 5 or of a pharmaceutically acceptable salt thereof for the preparation of a medicament for the treatment of Parkinson's disease, cephalalgias, depressions, hyperprolactinemias, hypertension and peripheral and cerebral vascular insufficiencies.